# FASCICULE DE BREVET EUROPEEN

(11) **EP 1 872 116 B1**
(45) Date de publication et mention de la délivrance du brevet: **12.10.2011**
(21) Numéro de dépôt: 06743351.6
(22) Date de dépôt: 19.04.2006
(51) Int. Cl.: G01N 27/416, G01N 33/18

(54) **PROCEDE UTILISANT UN CAPTEUR ELECTROCHIMIQUE ET ELECTRODES FORMANT CE CAPTEUR**
VERFAHREN UNTER VERWENDUNG EINES ELEKTROCHEMISCHEN SENSORS UND DIESEN SENSOR BILDENDER ELEKTRODEN
METHOD USING AN ELECTROCHEMICAL SENSOR AND ELECTRODES FORMING SAID SENSOR

(30) Priorité: 22.04.2005 EP 05103304
(43) Date de publication de la demande: 02.01.2008
(73) Titulaire: Adamant Technologies SA, 2300 La Chaux-de-Fonds (CH); ZÜLLIG AG, 9424 Rheineck (CH)
(72) Inventeur: SANTOLI, Eduardo, CH-2103 Noiraigue (CH); RYCHEN, Philippe, F-68640 Muespach Le Haut (FR); GOBET, Jean, CH-2035 Corcelles (CH); PFÄNDLER, Remo, CH-9403 Goldach (CH); BITSCHE, Paul, A-6845 Hohenems (AT)
(74) Mandataire: GLN
(86) Numéro de dépôt international: PCT/EP2006/061681
(87) Numéro de publication internationale: WO 2006/111550

(56) Documents cités:
- EP-A- 0 605 882
- US-A1- 2003 173 233
- DATABASE WPI Section EI, Week 200352 Derwent Publications Ltd., London, GB; Class S03, AN 2003-553300 XP002369703 & RU 2 207 558 A (URALS CHEM RES INST & EXPER WKS) 27 juin 2003 (2003-06-27) & RU 2 207 558 C2 (NAUCHNO-ISSLEDOVATEL'SKIJ KHIMICHESKIJ INSTITUT S) 27 juin 2003 (2003-06-27)
- PATENT ABSTRACTS OF JAPAN vol. 2000, no. 07, 29 septembre 2000 (2000-09-29) & JP 2000 093907 A (DAINIPPON SCREEN MFG CO LTD), 4 avril 2000 (2000-04-04)

## Description

### Domaine technique

La présente invention se rapporte aux capteurs électrochimiques destinés à mesurer la concentration d'une substance chimique dans un milieu aqueux. De tels dispositifs trouvent une application particulièrement intéressante, mais non exclusive, à la détection de la quantité d'oxygène dissous dans de l'eau, particulièrement dans les bassins des stations d'épuration.

L'invention concerne, plus particulièrement, un procédé d'auto-nettoyage d'un capteur électrochimique.

### Etat de la technique

Les capteurs électrochimiques du type mentionné ci-dessus comportent nécessairement une électrode de travail, une électrode de référence et une contre-électrode. On connaît également un autre type de tels capteurs qui comportent, en outre, une électrode, dite génératrice, et sa contre-électrode. L'ajout de ces deux dernières électrodes, dont l'effet est de créer des modifications de concentration d'espèces présentes en solution, permet de contrôler localement l'environnement de l'électrode de travail.

Par exemple, le pH de la solution peut être modifié localement par l'application d'un courant à l'électrode génératrice. Un courant cathodique entraînera la production d'ions OH- (le pH devenant alors plus basique) et, inversement, un courant anodique entraînera la production de ions H⁺ (le pH devenant alors plus acide). Une contre-électrode associée à l'électrode génératrice, une contre-électrode associée à l'électrode de travail et une électrode de référence sont nécessaires à la réalisation d'un capteur complet.

On comprendra aisément qu'il est particulièrement avantageux d'utiliser, comme électrode de travail, des électrodes de très petites dimensions, non seulement parce que cela permet de réduire l'espace entre l'électrode génératrice et l'électrode de travail, mais aussi parce que les effets de la turbulence du liquide au niveau de la cellule s'en trouvent minimisés. De telles électrodes de petites dimensions sont appelées indifféremment, dans la suite de la description, "micro-électrodes" ou "micro-disques", cette dernière appellation étant due au fait que les micro-électrodes sont le plus souvent de forme circulaire.

Le document WO 02/095387 décrit un capteur tel que mentionné précédemment et représenté à la figure 1. Il utilise un substrat électriquement conducteur 10, avantageusement réalisé en silicium dopé et dont la face inférieure est recouverte d'une couche de métallisation 11. Sa face supérieure est recouverte d'une couche de passivation 12 formée d'un empilement de deux sous-couches de SiO₂ et Si₃N₄, connu pour présenter une excellente stabilité en milieu aqueux.

La couche de passivation 12 est dotée d'ouvertures circulaires 13. Sa face supérieure et les ouvertures 13 sont recouvertes d'une couche conductrice 14 portant la référence 16 lorsqu'elle est sur la couche 12 et la référence 18 lorsqu'elle forme un micro-disque reposant dans l'une des ouvertures circulaires 13. La couche 14 est percée d'un réseau d'ouvertures annulaires 19 entourant chacune l'un des micro-disques 18.

La couche 16 forme l'électrode génératrice tandis que tous les micro-disques 18, électriquement connectés en parallèle les uns aux autres, constituent, ensemble, l'électrode de travail du système.

Les capteurs électrochimiques trouvent une application intéressante dans la mesure de la concentration en oxygène dissous dans les bassins de station d'épuration. En effet, les eaux usées sont traitées au moyen de bactéries qui sont très sensibles à cette concentration en oxygène dissous. L'information fournie par les capteurs permet de réguler un système d'aération du bassin pour que les conditions soient favorables aux bactéries. Cependant, les capteurs présents sur le marché ne donnent pas des résultats satisfaisants, notamment à cause de diverses contaminations et des matières organiques qui se déposent sur l'électrode, modifiant ainsi sa sensibilité. Avec les capteurs classiques, on a généralement recours, pour combattre la contamination, à des méthodes mécaniques du type air pulsé, jet d'eau sous pression, abrasion; méthodes qui présentent toutes beaucoup d'inconvénients.

Afin d'éliminer la contamination de la surface du capteur, la demande déposée sous le numéro EP 04 405039.1 propose d'utiliser une électrode génératrice en diamant. Lorsqu'elle est mise sous tension, cette électrode génère des espèces fortement oxydantes, telles que des radicaux hydroxyles, capables de brûler efficacement des matières organiques.

Cependant, l'effet obtenu par ces espèces oxydantes ne permet pas d'empêcher totalement toute contamination. Avec le temps, le capteur est progressivement affecté, ce qui détériore la précision de la mesure.

L'un des buts de la présente invention est donc de résoudre le problème susmentionné, en proposant un capteur dont la précision n'est pas tributaire des phénomènes de contamination.

### Divulgation de l'invention

De façon plus précise, l'invention concerne un procédé d'auto-nettoyage d'une électrode de travail d'un capteur électrochimique comportant, d'une part, une électrode de travail de type micro-disques et sa contre-électrode, et une électrode de référence reliées à un potentiostat, et, d'autre part, une électrode génératrice en diamant dopé au bore et sa contre-électrode reliées à une source de courant. L'ensemble de ces électrodes est, en outre, connecté à une électronique de contrôle et de mesure.

Avantageusement, le procédé comprend les étapes suivantes :
- application d'un courant anodique à l'électrode génératrice, et
- application d'un courant cathodique à l'électrode génératrice.

Le procédé d'auto-nettoyage peut précéder un procédé de mesure d'au moins une espèce dissoute dans un milieu aqueux. Dans ce cas, les électrodes de travail et de référence sont déconnectées de leur alimentation pendant l'étape d'auto-nettoyage.

### Brève description des dessins

D'autres détails de l'invention apparaîtront plus clairement à la lecture de la description qui suit, faite en référence au dessin annexé dans lequel :
- les figures 2 et 3 sont, respectivement, des vues de dessus et en coupe d'un système d'électrodes selon l'invention,
- les figures 4 et 5 illustrent la mise en ceuvre du système d'électrodes dans un dispositif de mesure selon l'invention, et
- la figure 6 représente l'évolution de la concentration en oxygène dissous en fonction du temps lors de la production d'oxygène par l'électrode génératrice, pour différentes conditions hydrodynamiques.

### Mode(s) de réalisation de l'invention

Dans la description ci-dessous, une tension négative se réfère à un potentiel cathodique et une tension positive se réfère à un potentiel anodique.

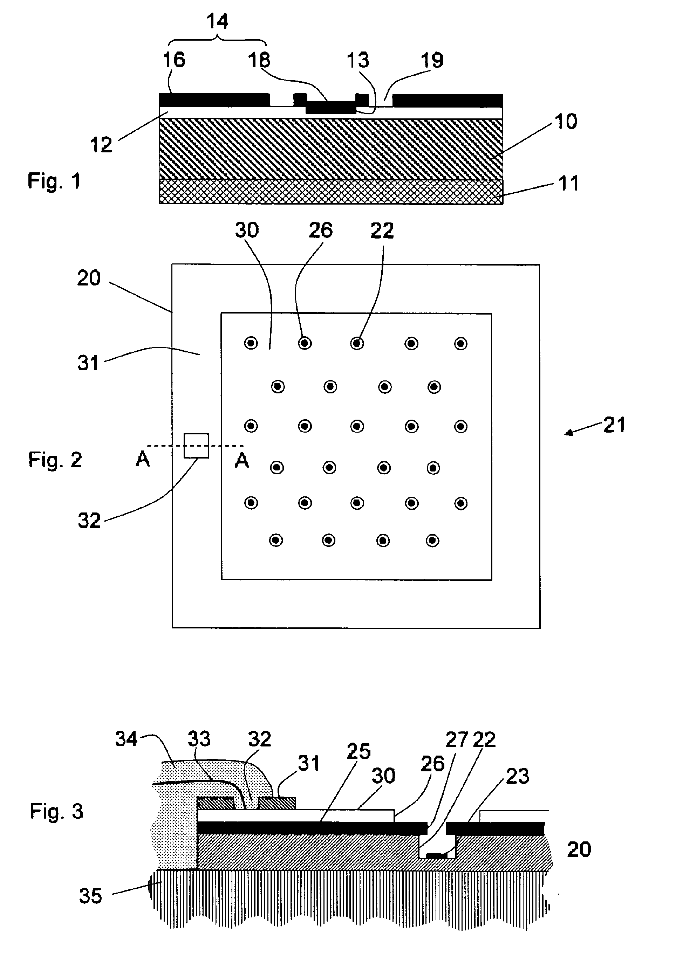

Les figures 2 et 3 représentent une puce 21. Elle possède un substrat électriquement conducteur 20 qui se présente sous la forme d'une plaque, par exemple carrée, typiquement, de 2 à 10 mm de côté et 0.5 mm d'épaisseur. Cette plaque est, avantageusement, réalisée en silicium rendu conducteur par dopage selon des techniques bien connues de l'homme du métier. Elle est posée sur un support 35, réalisé en polymère isolant.

Le substrat 20 est percé, sur sa face supérieure, d'un réseau régulier de cavités sensiblement cylindriques 22, d'axe perpendiculaire au plan du substrat. Typiquement, ces cavités ont un diamètre de 2 à 20 µm, une profondeur de 2 à 20 µm et sont espacées les unes des autres d'environ 40 à 400 µm.

Le fond de chaque cavité 22 est partiellement recouvert d'une métallisation 23 qui présente un diamètre inférieur de 0.5 à 5 µm à celui de la cavité 22. L'ensemble des métallisations 23 constitue l'électrode de travail du système. Avantageusement, un dépôt métallique peut être réalisé sur les métallisations 23 par croissance galvanique.

La face supérieure du substrat 20 est recouverte d'une couche isolante 25, dite de passivation, qui est formée, par exemple, d'un empilement de deux sous-couches de SiO₂ et Si₃N₄ et a une épaisseur d'environ 0.1 à 1 µm. Cette couche est percée d'un réseau régulier d'ouvertures traversantes circulaires 27 centrées sur les cavités 22, de diamètre inférieur à celui des cavités.

La structure qui vient d'être décrite est complétée par une électrode génératrice 30, qui peut être en diamant, disposée autour des électrodes de mesure selon l'enseignement du document WO 02/095387 et de la demande EP 04 405039.1.

Plus particulièrement, l'électrode 30 est formée d'une mince couche de diamant rendu conducteur par dopage, qui est percée d'ouvertures circulaires 26 de diamètre supérieur à celui des cavités 22 et disposée de manière à ce que chaque ouverture 26 soit concentrique à une micro-électrode. Avantageusement, le diamant utilisé pour former l'électrode est dopé au bore.

En variante, les électrodes de travail et génératrice peuvent être réalisées selon la structure représentée sur la figure 1.

Lorsque l'électrode 30 est mise sous tension, elle permet de générer des espèces fortement oxydantes telles que des radicaux hydroxyles. Comme expliqué dans l'introduction, ceux-ci peuvent avoir une action néfaste sur des revêtements en polymère 34, par exemple en silicone, généralement déposés sur les contacts électriques de l'électrode génératrice, plus précisément à l'interface entre le revêtement 34 et la couche de diamant 30 au contact du milieu.

Pour éviter ces effets, une couche de protection 31 est déposée sur l'électrode génératrice en diamant 30 préalablement au revêtement 34, de sorte qu'il n'existe plus d'interface directe entre le revêtement 34 et la couche de diamant 30. Cette couche de protection est réalisée en un matériau inorganique électriquement isolant, tel que du SiO₂ ou du Si₃N₄, qui, de plus, présentent une excellente stabilité en milieu aqueux. La couche 31 a une épaisseur comprise entre 0.1 et 0.5 µm. Elle est déposée et conformée selon les techniques connues de l'homme du métier.

Avantageusement, la couche de protection 31 est située à la périphérie de l'électrode 30 et forme un cadre de manière à recouvrir les éléments à protéger. Elle comprend des ouvertures 32 nécessaires à la connexion électrique avec l'électrode génératrice 30. Pour améliorer le contact électrique avec l'électrode 30, un cadre de contact métallique, non représenté, peut être déposé, par évaporation, sur la couche de protection 31 qui est munie de plusieurs ouvertures 32. Le cadre de contact peut aussi être réalisé séparément et ensuite collé à l'électrode 30, au niveau des ouvertures 32, par une colle conductrice.

Le revêtement en polymère 34 est ensuite déposé sur les contacts électriques et, s'il y en a un, sur le cadre de contact. Comme on peut le voir sur la figure 3, le revêtement déborde sur la couche de protection 31 et recouvre également les surfaces latérales de la puce 21.

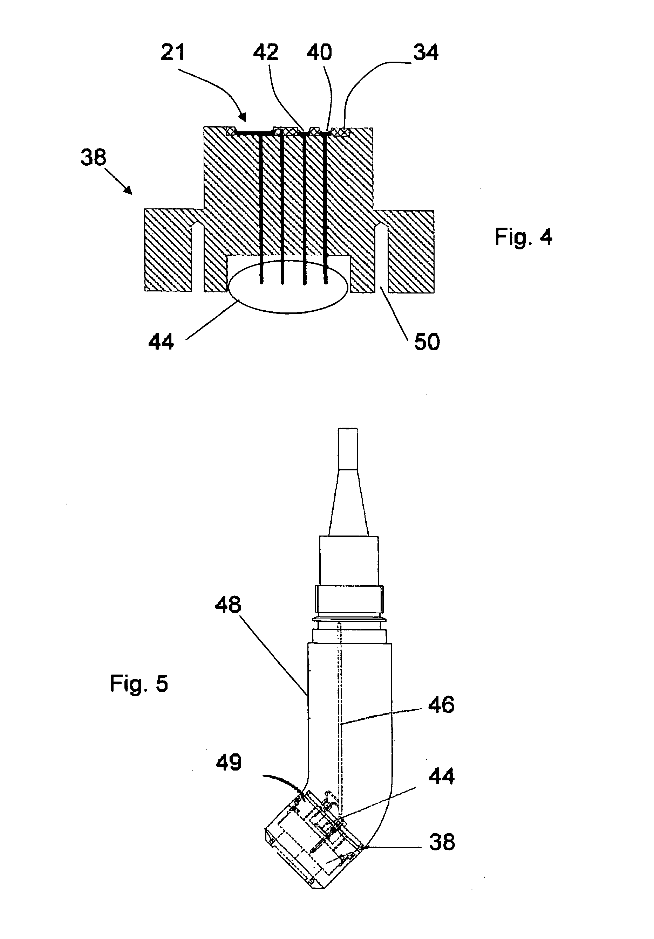

La puce 21 décrite ci-dessus comprend donc une électrode de travail (ensemble des métallisations 23) et une électrode génératrice 30. Elle est intégrée dans une tête de mesure 38 représentée, en coupe, à la figure 4. La tête de mesure 38 comporte, en outre, une contre-électrode pour l'électrode de travail 40 et une électrode de référence 42. La tête 38 est réalisée en un matériau non-métallique, du type polymère non conducteur. La tête 38 peut également comporter une contre-électrode pour l'électrode génératrice, mais on comprendra ci-après pourquoi il n'en figure pas dans le mode de réalisation décrit.

Les zones des électrodes destinées à être en contact avec le milieu à analyser affleurent à la surface de la tête de mesure 38 et sont situées à faible distance les unes des autres. Des éléments de connectique 44 traversent la tête de mesure pour relier les électrodes à l'électronique 46 de contrôle, de mesure et d'interface avec l'extérieur (affichage, enregistrement, etc.).

En résumé, on retiendra que l'électrode génératrice et sa contre-électrode sont branchées à une source de courant, tandis que l'électrode de travail et sa contre-électrode, d'une part, et l'électrode de référence, d'autre part, sont branchées à un potentiostat qui permet d'imposer une tension entre les électrodes et, ainsi, de mesurer, à une tension déterminée, le courant circulant entre l'électrode de travail et sa contre-électrode.

La contre-électrode de l'électrode de travail 40 est avantageusement réalisée en un matériau conducteur et chimiquement stable, tel que de l'acier inox, de l'or ou du platine. Sa surface est typiquement 100 fois supérieure à celle des micro-disques de l'électrode de travail 23. L'électrode de référence 42 est choisie en fonction de l'application visée. Pour une mesure de la concentration d'oxygène dans un milieu aqueux, une électrode du type Ag/AgCl est généralement préférée.

Comme indiqué ci-dessus pour la puce 21, une couche en matériau polymère 34 est déposée sur les bords des substrats des électrodes.

La tête de mesure 38 est montée de manière étanche à l'extrémité d'un corps de sonde 48, généralement de forme tubulaire, visible sur la figure 5. Pour assurer que les éléments de connectique 44 de la tête 38 sont positionnés correctement par rapport à l'électronique 46 qui prend place dans le corps de sonde, la tête 38 est dotée de trous de positionnement 50 qui coopèrent avec des ergots disposés dans le corps 48. Il est ainsi très facile, en cas de besoin, de remplacer la tête de mesure 38. La tête de mesure est ensuite fixée au corps de la sonde à l'aide d'une bague 49, qui est vissée sur le corps de la sonde de manière à assurer l'étanchéité de l'ensemble.

Dans une variante avantageuse illustrée, le corps de sonde 48 est réalisé en un matériau métallique, tel que de l'acier inox, et sert de contre-électrode pour l'électrode génératrice. Eventuellement, le corps 48 sert de contre-électrode pour l'électrode génératrice et/ou pour l'électrode de travail. Le corps 48 peut également comporter une partie non-métallique et une partie métallique, cette dernière étant utilisée comme contre-électrode.

L'extrémité du corps de sonde 48 qui reçoit la tête de mesure 38 est inclinée d'un angle compris entre 0 et 90°, préférablement compris entre 30 et 60°, préférablement de l'ordre de 45°, par rapport au reste du corps 48. Ce dernier étant plongé verticalement dans le liquide à analyser, l'angle que présente son extrémité permet d'améliorer les conditions hydrodynamiques autour de la tête de mesure 38, particulièrement lorsque le milieu est agité.

Un procédé d'utilisation du capteur électrochimique qui vient d'être décrit est expliqué ci-après, particulièrement en référence à une application à la mesure d'oxygène dissous dans un milieu aqueux.

La mesure ampérométrique de la concentration en oxygène dissous dans un milieu aqueux est réalisée en appliquant un potentiel de réduction adéquat entre l'électrode de travail et l'électrode de référence. Il se créé alors un courant de réduction proportionnel à la concentration d'oxygène dissous dans le liquide circulant entre l'électrode de travail et sa contre-électrode. On peut alors mesurer le courant dans l'électrode de travail, ce courant étant représentatif de la concentration en oxygène du milieu.

Une procédure d'auto-calibration, (non revendiquée) dont un exemple sera donné ci-dessous, permet de compenser efficacement les dérives dues à des pertes de sensibilité de l'électrode de travail. Cette procédure in situ est basée sur un changement de la concentration locale en oxygène dissous, obtenu au moyen de variations adaptées de la polarisation de l'électrode génératrice qui entraînent l'électrolyse de l'eau selon la réaction :

2H₂O → O₂ + 4H⁺ + 4e⁻

Cette réaction modifie localement la concentration en oxygène dissous de manière précise et indépendante de la teneur en oxygène du milieu environnant. En effet, la densité et la durée d'application du courant reçu par l'électrode génératrice permettent de contrôler la production d'oxygène.

La différence entre la réponse de l'électrode de travail avant et après la production d'oxygène par l'électrode génératrice permet de déduire un facteur de calibration. Ce dernier représente la sensibilité du capteur et fournit, pour un capteur donné, un coefficient de proportionnalité entre la concentration en oxygène du milieu à analyser et le courant effectivement mesuré entre l'électrode de travail et sa contre-électrode.

De manière générale, le temps pendant lequel la concentration en oxygène dissous est localement augmentée dépend des conditions d'agitation du milieu.

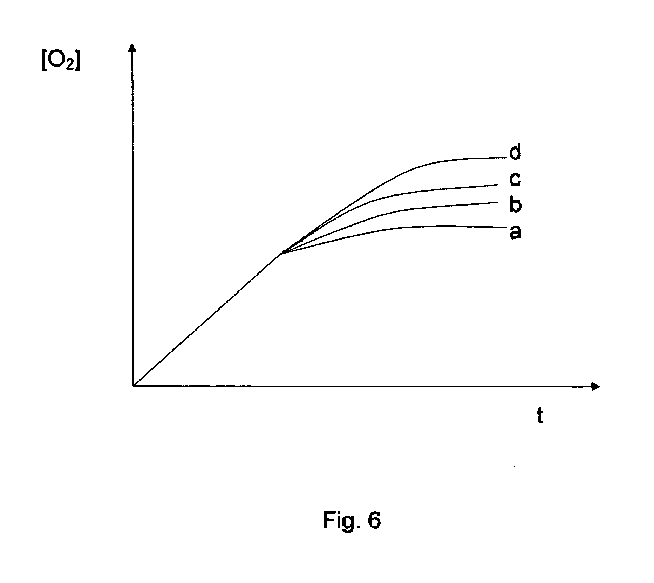

Comme le montre la figure 6, qui représente, sur les courbes a à d, l'évolution de la concentration d'oxygène en fonction du temps dans différentes conditions d'agitation, il a été constaté de manière surprenante que, pour une puce 21 dotée de micro-disques, la concentration croît de manière linéaire pendant les premiers instants de production d'oxygène, sans que les conditions hydrodynamiques n'aient d'influence. Cette phase linéaire dure environ 500 ms. Ainsi, si l'on n'applique le courant d'électrolyse à l'électrode génératrice que pendant une durée réduite, l'agitation du milieu ne perturbe pas la concentration en oxygène dans la zone de diffusion et il est alors possible de mesurer précisément le courant engendré par l'oxygène effectivement produit par l'électrode génératrice et de calculer un facteur de cal ibration.

Dans un milieu de faible conductivité, le champ électrique engendré par l'électrode génératrice peut perturber le comportement de l'électrode de référence au cours de l'auto-calibration si, lors de cette procédure, la mesure se fait simultanément avec le fonctionnement de l'électrode génératrice. Pour pallier cet inconvénient, le mesure d'auto-calibration est réalisée juste après l'arrêt de l'alimentation de l'électrode génératrice, sur un point ou en moyennant les valeurs obtenue sur une certaine plage, comme on le verra ci-dessous. Toutefois, une mesure peut être effectuée pendant l'application d'un courant à l'électrode génératrice.

Bien qu'une procédure d'auto-calibration permette de compenser les éventuelles dérives dues à l'encrassement des micro-électrodes, il est néanmoins intéressant de pouvoir conserver une bonne sensibilité de l'électrode de travail. Ceci est obtenu par une opération d'auto-nettoyage, réalisée in situ, et rendue possible grâce aux propriétés du diamant dopé au bore de l'électrode génératrice. Cette opération peut également être réalisée à titre préventif.

L'auto-nettoyage est obtenu par la conjonction de plusieurs effets causés par l'application à l'électrode de diamant de courants de polarité et de densité différentes. Tout d'abord, un courant anodique entraîne une production d'ion H⁺, ce qui acidifie le milieu aqueux proche de l'électrode. Ainsi, les dépôts de type tartre (carbonate de calcium, oxydes de magnésium) sont dissous. En outre, ce type de courant appliqué sur une électrode de diamant dopé au bore entraîne une production d'espèces fortement actives, telles que des radicaux hydroxyles, qui dégradent les dépôts organiques comme les bio-films, et possèdent une activité biocide.

De plus, l'application d'un courant cathodique entraîne la production d'ions OH⁻, ce qui rend basique le milieu aqueux proche de l'électrode. Ainsi, une alternance de courants anodique/cathodique fait fortement varier le pH à proximité de l'électrode de diamant, empêchant ainsi la formation d'un bio-film.

L'étape d'auto-nettoyage est appliquée à intervalles définis, de manière à ce que l'oxygène produit lors de l'application d'un courant anodique ne perturbe pas les mesures ultérieures.

Différentes procédures sont définies, comprenant chacune une suite détaillée de séquences faites dans un ordre déterminé de manière à réaliser les étapes d'auto-nettoyage, d'auto-calibration (non revendiquée) et de mesure (non revendiquée).

L'électronique de contrôle 46 mentionnée ci-dessus comprend, en outre, un microprocesseur programmé pour gérer :
- la durée des différentes étapes,
- les différences de potentiel entre l'électrode de travail et de référence,
- mesurer, sous une différence de potentiel déterminée et imposée par le potentiostat, le courant qui passe entre l'électrode de travail et sa contre-électrode,
- le courant appliqué entre l'électrode génératrice et sa contre-électrode, et
- la déconnexion des électrodes de travail et de référence au cours de la production d'oxygène de la phase d'auto-nettoyage.

Les autres éléments de l'électronique de contrôle 46 sont parfaitement accessibles à l'homme du métier et ne sont donc pas décrits ici en détail.

A intervalles définis, le courant circulant entre l'électrode de travail et sa contre-électrode est mesuré et la concentration en oxygène dissous est calculée au moyen du facteur de calibration déterminé préalablement. A titre d'exemple, une procédure type de mesure (non revendiquée), comprenant à titre facultatif une étape d'auto-nettoyage doux, comporte les séquences suivantes.
- Attente et auto-nettoyage doux :
   i. Les électrodes de travail et de référence sont déconnectées de leur alimentation, pendant une durée typiquement comprise entre 0 et 60s, plus particulièrement de 15s.
   ii. Un auto-nettoyage doux est obtenu en appliquant un courant anodique de densité typiquement comprise entre 0.5 et 2 mA/cm², plus particulièrement de 1 mA/cm², pendant une période variant de 0.5 à 5s, typiquement 1 s, débutant immédiatement après la déconnexion mentionnée au point i. La durée restant pour cette étape permet à l'oxygène produit au niveau de l'électrode génératrice de se disperser par diffusion dans le milieu.
- Activation de l'électrode de travail :
   i. Une activation anodique est obtenue en appliquant une tension typiquement comprise entre 200 et 1500mV, plus particulièrement de 500mV pendant une durée typiquement comprise entre 0.1 et 30s, plus particulièrement de 3s.
   ii. Une activation cathodique est obtenue en appliquant une tension typiquement comprise entre -200 et -1500mV, plus particulièrement de -1100mV pendant une durée typiquement comprise entre 0.1 et 10s, plus particulièrement de 1s.
- Mesure et calcul
   i. Une stabilisation est obtenue en appliquant à l'électrode de travail une tension typiquement comprise entre -500 et - 1200mV, plus particulièrement de -900mV pendant une durée typiquement comprise entre 0.1 et 60s, plus particulièrement de 8s.
   ii. Une mesure et une détermination du courant moyen circulant entre l'électrode de travail et sa contre-électrode sont réalisées sous une tension typiquement comprise entre -500 et -1300mV, plus particulièrement de -900mV, pendant une durée typiquement comprise entre 0.1 et 30s, plus particulièrement de 2s.
   iii. La concentration en oxygène dissous est calculée à partir du courant déterminé à l'étape précédente et au moyen du facteur de calibration prédéterminé selon la procédure donnée ci-dessous.

Une telle procédure de mesure est répétée à une fréquence typiquement de 0.5 à 5 fois par minute selon les valeurs des paramètres définis ci-dessus, plus particulièrement de 2 mesures par minutes.

Une procédure d'auto-calibration (non revendiquée) comprend, typiquement, les étapes suivantes :
- Attente :
   i. Les électrodes de travail et de référence sont déconnectées de leur alimentation, pendant une durée typiquement comprise entre 0 et 60s, plus particulièrement de 15s.
- Activation de l'électrode de travail :
   i. Une activation anodique est obtenue en appliquant une tension typiquement comprise entre 200 et 1500mV, plus particulièrement de 500mV pendant une durée typiquement comprise entre 0.1 et 30s, plus particulièrement de 3s.
   ii. Une activation cathodique est obtenue en appliquant une tension typiquement comprise entre -200 et -1500mV, plus particulièrement de -1100mV pendant une durée typiquement comprise entre 0.1 et 10s, plus particulièrement de 1 s.
- Mesure de la concentration en oxygène avant l'application d'un courant à l'électrode génératrice :
   i. Une stabilisation est obtenue en appliquant à l'électrode de travail une tension typiquement comprise entre -500 et - 1300mV, plus particulièrement de -900mV pendant une durée typiquement comprise entre 0.1 et 60s, plus particulièrement de 8s.
   ii. Une mesure et une détermination du courant moyen circulant entre l'électrode de travail et sa contre-électrode sont réalisées sous une tension typiquement comprise entre -500 et -1300mV, plus particulièrement de -900mV, pendant une durée typiquement comprise entre 0.1 et 30s, plus particulièrement de 2s.
- Application d'un courant d'auto-calibration à l'électrode génératrice de manière à produire une augmentation définie de la concentration locale en oxygène dissous:
   i. Un courant anodique d'une densité typiquement comprise entre 0.1 et 10 mA/cm², plus particulièrement de 1 mA/cm² est appliqué pendant une durée typiquement comprise entre 0.1 et 1 s plus particulièrement de 0.4s.
- Mesure de la concentration en oxygène après l'application d'un courant à l'électrode génératrice :
   i. Une stabilisation est obtenue en appliquant à l'électrode de travail une tension typiquement comprise entre -500 et - 1300mV, plus particulièrement de -900mV pendant une durée typiquement comprise entre 0.01 et 0.1 s, plus particulièrement de 0.04s.
   ii. Une mesure et une détermination du courant moyen circulant entre l'électrode de travail et sa contre-électrode sont réalisées sous une tension typiquement comprise entre -500 et -1300mV, plus particulièrement de -900mV, pendant une durée typiquement comprise entre 0.01 et 0.2s, plus particulièrement de 0.08s.
   iii. Un nouveau facteur de calibration de l'électrode de travail est calculé à partir de la différence des mesures avant et après application du courant d'auto-calibration sur l'électrode auxiliaire

Une telle procédure d'auto-calibration est répétée à une fréquence de 0.1 à 48 fois par jour, typiquement de 1 fois par jour.

Une procédure d'auto-nettoyage comprend, typiquement, les étapes suivantes :
i. application d'un courant anodique à l'électrode génératrice d'une densité typiquement comprise entre 0.5 et 100 mA/cm², plus particulièrement de 2 mA/cm² pendant une durée typiquement comprise entre 0.5 et 60s, plus particulièrement de 3s,
ii. application d'un courant cathodique à l'électrode génératrice d'une densité typiquement comprise entre 0.5 et 100 mA/cm², plus particulièrement de 2 mA/cm² pendant une durée typiquement comprise entre 0.5 et 60s, plus particulièrement de 3s, et
iii. répétition des deux étapes précédentes de 1 à 30 fois, plus particulièrement de 3 fois.

Une telle procédure d'auto-nettoyage est répétée à une fréquence de 10 à 200 fois par jour, typiquement de 100 fois par jour.

Ainsi sont proposées différentes procédures in situ particulièrement avantageuses d'auto-calibration (non revendiquée), d'auto-nettoyage et de mesure (non revendiquée) qui permettent de conserver une bonne précision d'un capteur électrochimique en évitant l'influence de toute contamination due au milieu de mesure. Par ailleurs, un capteur électrochimique peut permettre un auto-nettoyage, en évitant que les espèces oxydantes ne s'attaquent aux éléments constitutifs du capteur.

La présente invention ne se limite pas à un capteur électrochimique mesurant la concentration en oxygène dissous dans un milieu. En effet, il est possible d'établir une corrélation entre les courants mesurés pour différentes espèces analysées autre que l'oxygène, par exemple, du chlore ou de l'ozone.

## Revendications

1. Procédé d'auto-nettoyage d'une électrode de travail d'un capteur électrochimique comportant, d'une part, une électrode de travail de type micro-disques (23) et sa contre-électrode (40), et une électrode de référence (42) reliées à un potentiostat, et, d'autre part, une électrode génératrice (30) en diamant dopé au bore et sa contre-électrode (48) reliées à une source de courant, l'ensemble de ces électrodes étant, en outre, connecté à une électronique de contrôle et de mesure (46), ledit procédé comprenant les étapes suivantes :
- application d'un courant anodique à l'électrode génératrice, et
- application d'un courant cathodique à l'électrode génératrice.

2. Procédé d'auto-nettoyage selon la revendication 1, **caractérisé en ce que** lesdits courants anodique et cathodique sont appliqués avec une densité comprise entre 0.5 et 100 mA/cm2, plus particulièrement de 2 mA/cm2 pendant une durée comprise entre 0.5 et 60s, plus particulièrement de 3s,

3. Procédé d'auto-nettoyage selon l'une des revendications 1 et 2, **caractérisé en ce que** les étapes d'application d'un courant anodique et d'un courant cathodique sont répétées de 1 à 30 fois, plus particulièrement 3 fois.

4. Procédé d'auto-nettoyage selon l'une des revendications 1 à 3, **caractérisé en ce que** ledit capteur est destiné à mesurer la concentration en oxygène dissous dans un milieu aqueux.

5. Procédé d'auto-nettoyage selon l'une des revendications 1 à 4, destiné à précéder une étape de mesure d'au moins une espèce dissoute dans un milieu aqueux par un capteur tel que défini dans la revendication 1, **caractérisé en ce que** les électrodes de travail et de référence sont déconnectées de leur alimentation pendant l'étape d'auto-nettoyage.

6. Procédé d'auto-nettoyage selon la revendication 5, **caractérisé en ce que :**
- les électrodes de travail et de référence sont déconnectées de leur alimentation, pendant une durée comprise entre 0 et 60s, plus particulièrement de 15s,
- un auto-nettoyage est obtenu en appliquant un courant anodique de densité comprise entre 0.5 et 2 mA/cm², plus particulièrement de 1 mA/cm², pendant une période variant de 0.5 à 5s, plus particulièrement de 1 s, débutant immédiatement après ladite déconnexion.

7. Procédé d'auto-nettoyage selon l'une des revendications 5 et 6, **caractérisé en ce que** ladite espèce dissoute est l'oxygène.

## Claims

1. A self-cleaning method for a working electrode of an electrochemical sensor including a working electrode of the micro-disc type (23) and its counter electrode (40) and a reference electrode (42) connected to a potentiostat, on the one hand, and a generating electrode (30) in diamond doped with boron and its counter electrode (48) connected to a current source on the other hand, the set of these electrodes being further connected to an electronic control and measuring unit (46), said method comprising the following steps:
- applying an anode current to the generating electrode, and
- applying a cathode current to the generating electrode.

2. The self cleaning method according to claim 1, **characterized in that** said anode and cathode currents are applied with a density between 0.5 and 100 mA/cm², more particularly 2 mA/cm², for a time between 0.5 and 60 s, more particularly 3 s.

3. The self-cleaning method according to any of claims 1 and 2, **characterized in that** the steps for applying an anode current and a cathode current are repeated from 1 to 30 times, more particularly 3 times.

4. The self-cleaning method according to any of claims 1 to 3, **characterized** that said sensor is intended to measure the concentration of dissolved oxygen in an aqueous medium.

5. The self-cleaning method according to any of claims 1 to 4, intended to precede a step for measuring at least one dissolved species in an aqueous medium with a sensor as defined in claim 1, **characterized in that** the working and reference electrodes are disconnected from their power supply during the self-cleaning step.

6. The self-cleaning method according to claim 5, **characterized in that**:
- the working and reference electrodes are disconnected from their power supply, for a time between 0 and 60 s, more particularly 15 s,
- self-cleaning is obtained by applying an anode current with a density between 0.5 and 2 mA/cm², more particularly 1 mA/cm², for a period ranging from 0.5 to 5 s, more particularly 1 s, starting immediately after said disconnection.

7. The self-cleaning method according to any of claims 5 and 6, **characterized in that** said dissolved species is oxygen.

## Patentansprüche

1. Selbstreinigungsverfahren einer Arbeitselektrode eines elektrochemischen Sensors, das einerseits eine Arbeitselektrode vom Typ Mikroscheiben (23) und ihre Gegenelektrode (40) und eine Referenzelektrode (42), die mit einem Potentiostat verbunden sind, und andererseits eine Generatorelektrode (30) aus bordotiertem Diamant und ihre Gegenelektrode (48), die mit einer Stromquelle verbunden sind, aufweist, wobei die Gruppe dieser Elektroden weiterhin mit einer Kontroll- und Messelektronik (46) verbunden ist, wobei das Verfahren die folgenden Schritte umfasst:
- Anlegen eines Anodenstroms an die Generatorelektrode, und
- Anlegen eines Kathodenstroms an die Generatorelektrode.

2. Selbstreinigungsverfahren nach Anspruch 1, **dadurch gekennzeichnet, dass** der Anoden- und Kathodenstrom mit einer Dichte zwischen 0,5 und 100 mA/cm² inklusive, insbesondere von 2 mA/cm² während einer Dauer zwischen 0,5 und 60 s inklusive, insbesondere von 3 s, angelegt sind.

3. Selbstreinigungsverfahren nach einem der Ansprüche 1 und 2, **dadurch gekennzeichnet, dass** die Schritte des Anlegens eines Anodenstroms und eines Kathodenstroms 1 bis 30 Mal, insbesondere 3 Mal, wiederholt werden.

4. Selbstreinigungsverfahren nach einem der Ansprüche 1 bis 3, **dadurch gekennzeichnet, dass** der Sensor zum Messen der Konzentration des in einem wässrigen Milieu gelösten Sauerstoffs bestimmt ist.

5. Selbstreinigungsverfahren nach einem der Ansprüche 1 bis 4, das dazu bestimmt ist, vor einem Schritt des Messens von mindestens einer in einem wässrigen Milieu gelösten Substanz durch einen wie in Anspruch 1 definierten Sensor zu erfolgen, **dadurch gekennzeichnet, dass** die Arbeits- und Referenzelektroden während des Selbstreinigungsschritts von ihrer Versorgung getrennt sind.

6. Selbstreinigungsverfahren nach Anspruch 5, **dadurch gekennzeichnet, dass**
- die Arbeits- und Referenzelektroden von ihrer Versorgung während einer Dauer zwischen 0 und 60 s inklusive, insbesondere von 15 s, getrennt sind,
- eine Selbstreinigung durch Anlegen eines Anodenstroms einer Dichte zwischen 0,5 und 2 mA/cm² inklusive, insbesondere von 1 mA/cm², während eines Zeitraums, der von 0,5 bis 5 s schwankt, insbesondere 1 s, wobei er sofort nach der Trennung beginnt, erfolgt.

7. Selbstreinigungsverfahren nach einem der Ansprüche 5 und 6, **dadurch gekennzeichnet, dass** die gelöste Substanz Sauerstoff ist.
